# EUROPÄISCHE PATENTANMELDUNG

(11) **EP 2 774 911 A1**
(43) Veröffentlichungstag der Anmeldung: **10.09.2014**
(21) Anmeldenummer: 13158020.1
(22) Anmeldetag: 06.03.2013
(51) Int. Cl.: C07C 209/26, C07C 211/29

(54) **Verfahren zur Herstellung von Halogen-N,N-dimethylbenzylaminen**

(71) Anmelder: LANXESS Deutschland GmbH, 50569 Köln (DE)
(72) Erfinder: Schulze Tilling, Andreas, 42799 Leichlingen (DE); Peters, Lars, 51371 Leverkusen (DE); Stirner, Wolfgang, 51467 Bergisch Gladbach (DE)

(57) **Zusammenfassung**

Die Erfindung betrifft ein Verfahren zur Herstellung von Halogen-N,N-dimethylbenzylaminen mit Halogen = Chlor oder Brom, vorzugsweise Chlor-N,N-dimethylbenzylaminen, vorzugsweise ortho-Chlor-N,N-dimethylbenzylamin (o-Cl-DMBA), durch reduktive Aminierung in Abwesenheit von Schwefel.

## Beschreibung

Die Erfindung betrifft ein Verfahren zur Herstellung von Halogen-N,N-dimethylbenzylaminen mit Halogen = Chlor oder Brom, vorzugsweise Chlor-N,N-dimethylbenzylamin (Cl-DMBA) durch reduktive Aminierung. Das erfindungsgemäße Verfahren hat dabei den Vorteil, dass es in Abwesenheit von Schwefel durchgeführt werden kann.

Es ist bekannt, dass aus einem Aldehyd und einem sekundären Amin zunächst ein Halbaminal gebildet wird (Aminierung), welches unter Abspaltung von Wasser auch mit einem weiteren Molekül des Amins zum Aminal weiterreagieren kann.

Alle genannten Umsetzungsprodukte (Halbaminal bzw. Aminal) können katalytisch zu den entsprechenden Aminen hydriert werden. Diese Reduktion ist beispielsweise beschrieben in Houben-Weyl, Methoden der Organischen Chemie, 4. Auflage, Band IV/1c (1980), S. 127/128, 239/240 u. 436. Solche katalytischen Hydrierungen sollen auch unter Erhaltung von Halogen möglich sein. Jedoch ist dieses Ergebnis auf die Benutzung von Palladium-Katalysatoren beschränkt, und es wird weiter darauf hingewiesen, dass man vorteilhafterweise mit desaktiviertem Katalysator und bei niederen Temperaturen arbeitet (Houben-Weyl, Methoden der Organischen Chemie, 4. Auflage, Band IV/1c (1980), Seite 240). Auch unter Verwendung anderer Katalysatoren, wie Platin oder Raney-Nickel soll Halogen erhalten bleiben. Die an den zitieren Stellen angegebenen speziellen Hydrierungen stellen jedoch keine systematische Untersuchung dar (Houben-Weyl, Methoden der Organischen Chemie, 4. Auflage, Band IV/1c (1980), Seite 436, Absatz 3) und zeigen zum Teil überaus mäßige Ausbeuten, wie im Falle des p-Chlorbenzyl-methylketons, welches nur in 10 % der theoretischen Ausbeute in das zugehörige Amin übergeführt werden kann (Houben-Weyl, Methoden der Organischen Chemie, 4. Auflage, Band IV/1c (1980), Seite 436, unten). Insbesondere bei der Herstellung stark basischer Amine muss mit Nebenprodukten gerechnet werden (Houben-Weyl, Methoden der Organischen Chemie, 4. Auflage, Band IV/1c (1980), Seite 240, Absatz 2).

Aus EP-A-0 355 351 ist die katalytische Hydrierung in Gegenwart von Schwefel in Form organischer Schwefelverbindungen bekannt. Schwefelverbindungen haben jedoch den Nachteil, dass diese genau zudosiert werden müssen, da diese bei einer Fehl-/Überdosierung den Katalysator vergiften. Schwefelverbindungen haben zudem negative Auswirkungen auf das Endprodukt, insbesondere auf Chlor-N,N-dimethylbenzylamin, so dass diese aufwändig abgetrennt werden müssen.

Bei den Verfahren gemäß Stand der Technik werden entweder teure Edelmetallkatalysatoren verwendet, nur geringe Ausbeuten erreicht, oder die zugesetzten Additive müssen aufwändig abgetrennt werden, was diese Verfahren häufig unwirtschaftlich macht.

Vor dem Hintergrund des vorstehend genannten Standes der Technik bestand daher die Aufgabe, ein besonders wirtschaftliches Verfahren bereitzustellen, das die Herstellung von Halogen-N,N-dimethylbenzylamine mit Halogen = Cl oder Br, vorzugsweise Chlor-N,N-dimethylbenzylamin, besonders bevorzugt ortho-Chlor-N,N-dimethylbenzylamin (o-Cl-DMBA), in hohen Ausbeuten und in Abwesenheit von Schwefel und schwefelhaltigen Verbindungen oder anderer Katalysatorgifte bzw. Additive, die die Katalysatoraktivität vermindern, erlaubt.

Es wurde überraschend gefunden, dass die Nachteile des Standes der Technik vermieden werden können und Halogen-N,N-dimethylbenzylamine mit Halogen = Cl oder Br, vorzugsweise Cl, in hoher Ausbeute und in Abwesenheit von Schwefel hergestellt werden können, wenn bei der reduktiven Aminierung ein bestimmtes molares Verhältnis von Halogen-Benzaldehyd mit Halogen = Cl oder Br zu Dimethylamin eingehalten wird.

Gegenstand der vorliegenden Erfindung ist daher ein Verfahren zur Herstellung von Halogen-N,N-dimethylbenzylaminen mit Halogen = Cl oder Br, vorzugsweise o-, m- oder p-Chlor-N,N-dimethylbenzylamin, ganz besonders bevorzugt o-Chlor-N,N-dimethylbenzylamin, durch reduktive Aminierung von Halogenbenzaldehyd mit Halogen = Chlor oder Brom mit Dimethylamin in Gegenwart eines Katalysators, vorzugsweise eines Ni-haltigen und/oder Co-haltigen Katalysators, wobei das molare Verhältnis von Halogen-Benzaldehyd mit Halogen = Cl, Br zu Dimethylamin 1:>1,5 beträgt. Halogen kann dabei in o-(= ortho), m-(= meta) oder p-(= para)-Position, vorzugsweise in o-Position sein.

Besonders bevorzugt ist ein molares Verhältnis von Halogen-Benzaldehyd zu Dimethylamin von 1:1,51 - 10, ganz besonders bevorzugt von 1: 2-5.

Bei den eingesetzten Halogenbenzaldehyden handelt es sich um handelsübliche Verbindungen, die z.B. unter den Namen 2-Chlorbenzaldehyd bei der Merck Millipore GmbH oder 3-Chlor- bzw. 4-Chlorbenzaldehyd bei der Alfa Aesar GmbH & Co KG kommerziell erhältlich sind.

Bei dem eingesetzten Dimethylamin handelt es sich um eine handelsübliche Verbindung, die unter den Namen Dimethylamin wasserfrei 2.8 (99,8%ig) bei GHC Gerling, Holz und Co. Handels GmbH, kommerziell erhältlich ist.

Als Katalysatoren werden erfindungsgemäß Palladium, Platin, Ruthenium sowie Nickel und/oder Kobalt eingesetzt. Bevorzugt sind Ni- und/oder Co-Katalysatoren, erzeugt durch Auslaugen von Ni- oder Co-Legierungen, Ni oder Co auf Trägern, in Form von Skelettkatalysatoren, elementarem Ni(Co)-Schwamm, als Ni-Oxid, Co-Oxid, Raney-Nickel, Raney-Kobalt. Träger sind beispielsweise SiO₂, Al₂O₃, Bims, Kohle und andere dem Fachmann bekannte Träger. Besonders bevorzugt sind dabei Katalysatoren, wie Ni- oder Co-Katalysatoren, erzeugt durch Auslaugen von Ni oder Co-Legierungen, die als Bestandteil der Legierung unter anderem Legierung von Nickel, Kobalt, Nickel-Eisen, Nickel-Kobalt oder Nickel-Eisen-Kobalt in wasserfreier oder auch wasserfeuchter oder lösungsmittelfeuchter Form eingesetzt. Die Ni- und Co-haltigen Katalysatoren können auch gemeinsam eingesetzt werden.

Unter Skelettkatalysator werden solche Katalysatoren verstanden, die durch Auslaugen mit starken Basen, vorzugsweise mit konzentrierter Natronlauge entstanden sind. Die dadurch entstehende poröse Struktur wird auch Skelettstruktur genannt.

Der Katalysator wird dabei vorzugsweise in einer Menge von 0,1-25 Gew.-%, bevorzugt 1,5-12,5 Gew.-%, bezogen auf das zu hydrierende Substrat, eingesetzt.

In besonders bevorzugter Weise werden Ni-haltige Skelett-Katalysatoren eingesetzt. Bei den Katalysatoren handelt es sich um handelsübliche Katalysatoren, wie sie z.B. bei der Firma H.C. Starck GmbH hergestellt werden.

Die Reaktion kann dabei in Anwesenheit oder aber auch in Abwesenheit von Lösungsmitteln erfolgen, wobei die Abwesenheit von Lösungsmitteln bevorzugt ist.

Als Lösungsmitteln können Alkohole, vorzugsweise Methanol, Ethanol, Isopropanol, Butanol, aliphatische oder aromatische Kohlenwasserstoffe, vorzugsweise Toluol, Xylol, Cyclohexan, Isooctan und ähnliche, Ether, wie Tetrahydrofuran, Dioxan oder Methyl-tert.-butylester, Ester wie Ethylacetat und schließlich das Reaktionsprodukt selbst, sofern es bei der Reaktionstemperatur flüssig ist, eingesetzt werden. Ein Gehalt an Wasser (z.B. bis zu 20 Gew.-%) stört nicht, insbesondere, wenn das Reaktionsmedium mit Wasser mischbar ist.

Die Hydrierung wird vorzugsweise bei 30-250°C, bevorzugt bei 50-150°C, und einem H₂-Druck von 5-250 bar, bevorzugt 10-220 bar, durchgeführt.

Bei dem für die Hydrierung eingesetzten Wasserstoff handelt es sich vorzugsweise um kommerziell erhältlichen Wasserstoff.

Im Allgemeinen wird das erfindungsgemäße Verfahren so durchgeführt, dass man Dimethylamin und Halogen-N,N-dimethylbenzylamin, gegebenenfalls das Lösungsmittel und den Katalysator, in einem Hydrierautoklaven vorlegt und nach Verschließen des Reaktors die Luft mit Inertgas, vorzugsweise Argon und Stickstoff, versetzt, und anschließend das Inertgas mit Wasserstoff verdrängt. Mit Wasserstoff wird anschließend der gewünschte Druck eingestellt. In einer bevorzugten Ausführungsform der Erfindung wird solange nachgerührt bis der Druck des nachgeführten Wasserstoffs konstant bleibt. Nach beendeter Reaktion (Wasserstoffaufnahme) wird das Reaktionsgefäß zunächst entspannt, vorzugsweise auf Raumtemperatur abgekühlt und entleert; der Katalysator wird abfiltriert und kann ohne Aufbereitung wiederverwendet werden.

Das Verfahren kann sowohl diskontinuierlich als auch kontinuierlich, vorzugsweise in einem Festbettreaktor, durchgeführt werden.

In einer bevorzugten Ausführungsform wird bei der reduktiven Aminierung zusätzlich Essigsäure zugesetzt. Dabei handelt es sich um handelsübliche Essigsäure, in einem Konzentrationsbereich von 0,1 bis 10%, die z.B. bei der Firma Merck Millipore erhältlich ist. In einer bevorzugten Ausführungsform der Erfindung beträgt der Gehalt an Essigsäure vorzugsweise 0,5 - 5 Gew.%, besonders bevorzugt 0,5 - 4 Gew.%.

In einer weiteren bevorzugten Ausführungsform der Erfindung wird das Verfahren in Abwesenheit von Schwefel durchgeführt.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Die nachfolgenden Beispiele dienen der Erläuterung der Erfindung, ohne dabei limitierend zu wirken.

### Beispiele:

In einem 0,7 L VA Autoklav wurden 150 g (1,06 mol) o-Chlorbenzaldehyd, 3 g (0,05 mol) Eisessig und 6 g Nickelkatalysator (ein Katalysator der Firma HC Starck GmbH) zusammen vorgelegt. Der Autoklav wurde geschlossen, bei Raumtemperatur wurden 213,6 g (4,74 mol) Dimethylamin aufgedrückt. Mit Wasserstoff wurde anschließend ein Druck von 50 bar eingestellt und auf 100°C aufgeheizt. Nach Erreichen der Zieltemperatur steigerte man den Innendruck mit Wasserstoff auf 200 bar und hielt diesen. Sobald der Druck des nachgeführten Wasserstoffs konstant blieb, wurde 30 min bei 100°C nachgerührt. Nach beendeter Wasserstoffaufnahme und Abkühlen auf Raumtemperatur wurde die Reaktionsmischung ausgenommen (294,5 g Rohprodukt, ohne Aufarbeitung) und feste Anteile an Katalysator und Nebenprodukten per Filtration abgetrennt. Das Produkt wird gaschromatographisch analysiert und darauf basierend die Ausbeute bestimmt.

| **Versuch** | **Lösungsmittel** | **E/V** | **Parameter** | **molares Verhältnis o-Cl-Benzaldehyd : Dimethylamin** | **Ausbeute o-Cl-N,N-dimethyl-benzylamin** |
|---|---|---|---|---|---|
| 1 | ohne | V | 50bar, 150°C, | 1 : 1,5 | 0,7 |
| 2 | ohne | E | 50bar, 150°C | 1 : 4,5 | 55,5 |
| 3 | ohne | V | 150bar, 150°C | 1 : 1,5 | 50,7 |
| 4 | ohne | E | 150bar, 150°C | 1 : 4,5 | 73,3 |
| 5 | ohne | V | 50bar, 90°C | 1 : 1,5 | 78,1 |
| 6 | ohne | E | 50bar, 90°C | 1 : 4,5 | 81,9 |
| 7 | ohne | V | 150bar, 90°C | 1 : 1,5 | 81,1 |
| 8 | ohne | E | 150bar, 90°C | 1 : 4,5 | 90,6 |
| 9 | ohne | E | 100bar, 120°C | 1 : 3 | 85,5 |

| | | | | | |
|---|---|---|---|---|---|
| V = vergleich, E = erfindungsgemäß | | | | | |

Aus den Beispielen ist klar ersichtlich, dass bei einem molaren Verhältnis von o-Chlorbenzaldehyd zu Dimethylamin von 1 : > 1,5 deutlich bessere Ausbeuten erzielt werden.

## Patentansprüche

1. Verfahren zur Herstellung von Halogen-N,N-dimethylbenzylaminen mit Halogen = Chlor oder Brom durch reduktive Aminierung von Halogenbenzaldehyd mit Halogen = Chlor oder Brom mit Dimethylamin in Gegenwart eines Katalysators, **dadurch gekennzeichnet, dass** Halogenbenzaldehyd mit Dimethylamin in einem molaren Verhältnis von 1 : > 1,5, bevorzugt 1: 1,51 - 10, besonders bevorzugt von 1:2 - 5, umgesetzt wird.

2. Verfahren nach Anspruch 1, **dadurch gekennzeichnet, dass** Halogen für Chlor steht.

3. Verfahren nach Anspruch 1 oder 2, **dadurch gekennzeichnet, dass** als Katalysator Palladium, Platin, Ruthenium, Nickel- oder Cobalt oder Ni-haltige und/oder Co-haltigen Katalysatoren eingesetzt werden.

4. Verfahren nach einem der Ansprüche 1 bis 3, **dadurch gekennzeichnet, dass** als Katalysatoren Ni- oder Co-Katalysatoren, erzeugt durch Auslaugen von Ni oder Co-Legierungen, Ni oder Co auf Trägern ausgewählt aus der Gruppe SiO₂, Al₂O₃, Bims, Kohle in Form von Skelettkatalysatoren, elementarem Ni(Co)-Schwamm, als Ni-Oxid, Co-oxid, Raney-Nickel, Raney-Kobalt verwendet werden.

5. Verfahren nach einem der Ansprüche 1 bis 4, **dadurch gekennzeichnet, dass** dieses bei einer Temperatur von 30-250°C, bevorzugt bei 50-150°C, durchgeführt wird.

6. Verfahren nach einem der Ansprüche 1 bis 5, **dadurch gekennzeichnet, dass** dieses bei einem H₂-Druck von 5-250 bar, bevorzugt 10-220 bar, durchgeführt wird.

7. Verfahren nach einem der Ansprüche 1 bis 6, **dadurch gekennzeichnet, dass** dieses bei einer Temperatur von 30-250°C, bevorzugt bei 50-150°C und einem H₂-Druck von 5-250 bar, bevorzugt 10-220 bar, durchgeführt wird.

8. Verfahren nach einem der Ansprüche 1 bis 7, **dadurch gekennzeichnet, dass** der Katalysator in Mengen von 0,1-25 Gew.-%, bevorzugt 1,5-12,5 Gew.-%, bezogen auf das zu hydrierende Substrat, eingesetzt wird.

9. Verfahren nach einem der Ansprüche 1 bis 8, **dadurch gekennzeichnet, dass** zusätzlich Essigsäure zugesetzt wird.

10. Verfahren nach einem der Ansprüche 1 bis 9, **dadurch gekennzeichnet, dass** die Essigsäure im Bereich von 0,1 - 10 Gew.%, bevorzugt 0,1 - 5 Gew.%, besonders bevorzugt 0,5 - 4 Gew.%, eingesetzt wird.

11. Verfahren nach einem der Ansprüche 1 bis 10, **dadurch gekennzeichnet, dass** dieses ohne Lösungsmittel durchgeführt wird.

12. Verfahren nach einem der Ansprüche 1 bis 10, **dadurch gekennzeichnet, dass** dieses in Anwesenheit mindestens eins Lösungsmittels, ausgewählt aus der Gruppe Methanol, Ethanol, Isopropanol, Butanol, Toluol, Xylol, Cyclohexan, Isooctan, Tetrahydrofuran, Dioxan oder Methyl-tert.-butylester oder Ethylacetat, durchgeführt wird.

13. Verfahren nach einem der Ansprüche 1 bis 12, **dadurch gekennzeichnet, dass** dieses in Abwesenheit von Schwefel durchgeführt wird.
